Europäisches Patentamt

European Patent Office

Office européen des brevets

(19)

(11) Veröffentlichungsnummer: **0 003 611**
**A2**

(12) # EUROPÄISCHE PATENTANMELDUNG

(21) Anmeldenummer: **79100428.6**

(22) Anmeldetag: **13.02.79**

(51) Int. Cl.²: **A 61 F 5/01**

(30) Priorität: **14.02.78 DE 2806173**

(43) Veröffentlichungstag der Anmeldung: **22.08.79**
**Patentblatt 79/17**

(84) Benannte Vertragsstaaten: **BE CH DE FR GB IT NL**

(71) Anmelder: **A. Nattermann & Cie. GmbH,
Nattermannallee 1, D-5000 Köln 30 (DE)**

(72) Erfinder: **Prahl, Gertraud, Nutzfelder Weg 13, D-2127
Rullstorf (DE)**

(74) Vertreter: **Wuesthoff, Franz, Dr.-Ing. et al,
Patentanwälte Wuesthoff -v. Pechmann-Behrens-
Goetz Schweigerstrasse 2, D-8000 München 90 (DE)**

(54) **Spreizvorrichtung zum Behandeln von Hüft-Dysplasien.**

(57) Bei einer Spreizvorrichtung zum Behandeln von Hüft-Dysplasien ist eine im Bereich des Kreuzbeins an den Rücken eines Patienten anlegbaren Rückenstütze (1) vorgesehen, von der sich zwei Seitenteile (2) nach vorne erstrecken. Mit den Seitenteilen (2) ist über je einen nach vorne innen verlaufenden Bogen (3), der zwischen Becken und Oberschenkel an den Patienten anzulegen ist, je eine blattfederartige Oberschenkelschiene (5) verbunden. Die Oberschenkelschienen (5) sind diagonal von vorne oben nach hinten unten an die Innenseiten der Oberschenkel anzulegen. Am Ende der Oberschenkelschienen (5) ist je eine Stützschale (6) ausgebildet. Die Stützschalen (6) sind an oder über der Kniekehle an je einen Oberschenkel des Patienten anzulegen. Die Spreizvorrichtung ist als insgesamt blattfederartige, den Damm- und Gesäßbereich des Patienten freilassende Spreizspange gestaltet.

PATENTANWÄLTE
## WUESTHOFF - v. PECHMANN - BEHRENS - GOETZ

PROFESSIONAL REPRESENTATIVES BEFORE THE EUROPEAN PATENT OFFICE
MANDATAIRES AGRÉÉS PRÈS L'OFFICE EUROPÉEN DES BREVETS

D-8000 MÜNCHEN 90
SCHWEIGERSTRASSE 2
TELEFON: (089) 66 20 51
TELEGRAMM: PROTECTPATENT
TELEX: 5 24 070

B e s c h r e i b u n g

zur Patentanmeldung der

A. Nattermann & Cie. GmbH,
Nattermannallee 1, 5000 Köln-30

EP-51 861

Spreizvorrichtung zum Behandeln von Hüft-Dysplasien

Die Erfindung betrifft eine Spreizvorrichtung zum Behandeln von Hüft-Dysplasien mit einer im Bereich des Kreuzbeins an den Rücken eines Patienten anlegbaren Rückenstütze, von der sich zwei Seitenteile nach vorne erstrecken, mit denen je eine blattfederartige Oberschenkelschiene verbunden ist, an deren Ende je eine Stützschale befestigt ist, die an oder über der Kniekehle an je einen Oberschenkel des Patienten anzulegen ist.

Hüft-Dysplasien oder Dysplasien der Hüftgelenke, die sich mit solchen Vorrichtungen behandeln lassen, treten nicht selten bei neugeborenen Kindern auf und bestehen meist darin, daß bei den Hüftgelenken das Pfannendach zu steil ausgebildet ist und der Hüftkopf nicht im Mittelpunkt der Pfanne steht, ohne daß damit eine Luxation oder Subluxation verbunden zu sein braucht. Solche schlecht angelegten Hüftgelenke bedürfen einer Behandlung, die den Hüftkopf in das Pfannenzentrum orientiert und einen axial wirkenden, stimulierenden Druck ausübt, damit die Gelenkbildung begünstigt wird. Dies läßt sich dadurch erreichen, daß die Beine in eine Abspreizposition gebracht werden, in der sich der Hüftkopf infolge der Anlage der Muskulatur automatisch in das Zentrum der Hüftpfanne nach unten orientiert. Die Behandlung mit einer Spreizvorrichtung wird meist schon im Säuglings- oder Kleinkindalter eingeleitet, bevor das Kind beginnt, zu stehen und zu laufen. Die Spreizvorrichtung soll die Bewegungen des Kindes nicht erheblich einschränken, damit die Muskelentwicklung nicht gehemmt wird.

Bei einer bekannten Spreizvorrichtung der eingangs beschriebenen Gattung gehören Rückenstütze und Seitenteile zu einem Beckenkorb mit Oberschenkelöffnungen, dessen Vorderseite von einem Spreizsteg mit einem nach oben ragenden Fortsatz gebildet ist, der im Bereich seines oberen Endes durch Halterungseinrichtungen mit beiden Seitenteilen oberhalb der Oberschenkelöffnungen verbunden ist. Die blattfederartigen Oberschenkelschienen sind oberhalb der Oberschenkelöffnungen an je einem Seitenteil schwenkbar gelagert und schließen in Ruhestellung mit der Sagittal- oder Symmetrieebene des Beckenkorbes einen Winkel von je 30 bis 40° ein; jede Oberschenkelschiene verläuft deshalb parallel zum zugehörigen Oberschenkel an dessen Außenseite. Die Stützschalen sind an den Oberschenkelschienen in deren Längsrichtung einstellbar befestigt und weisen je einen schenkelumspannenden Gurt auf.

Der angestrebte Behandlungserfolg läßt sich mit einer solchen bekannten Spreizvorrichtung nur dann erzielen, wenn die folgenden Bedingungen eingehalten werden:

a) Der Beckenkorb muß mit einem Hüftgurt und durch Festziehen der beschriebenen Halterungseinrichtungen zwischen dem nach oben ragenden Fortsatz des Spreizsteges und den beiden Seitenteilen derart am Kind festgelegt sein, daß dieses sich im Beckenkorb nicht seitlich verschieben kann. Andernfalls könnte das Kind eine Lage einnehmen, bei der die erforderliche Abspreizung nur bei dem einen Oberschenkel sichergestellt ist, während der andere fast in die normale Anspreizposition zurückgeführt werden könnte.

b) Die Stützschalen müssen so eingestellt sein, daß die Kniekehlen exakt erreicht werden, und die zugehörigen Gurte müssen eng um die Oberschenkel herumgelegt werden. Andernfalls hätte

- 3 -

das Kind die Möglichkeit, durch Anspreizen der Oberschenkel den Hüftkopf beider Hüftgelenke wieder an den oberen Pfannenrand herauszudrücken, wodurch sich eine für den Behandlungserfolg ungünstige mechanische Lastverteilung ergäbe.

Diese Bedingungen werden von den kindlichen Patienten meist schon nach kurzer Zeit als lästig empfunden und rufen deshalb den Wunsch des Kindes wach, sich von wenigstens einem Teil der schenkel- und leibumspannenden Gurte zu befreien, was größeren Kindern auch bald gelingt, so daß auch von daher der Behandlungserfolg infragegestellt ist. Der bekannte Beckenkorb erschwert auch die Körperpflege des Kindes.

Der Erfindung liegt deshalb die Aufgabe zugrunde, eine Spreizvorrichtung der eingangs beschriebenen Gattung derart weiterzubilden, daß sie bei möglichst geringer Belästigung des Patienten Gewähr dafür bietet, daß eine für den Behandlungserfolg günstige Abspreizstellung und Belastung der Oberschenkel eingehalten wird.

Die Aufgabe ist erfindungsgemäß dadurch gelöst, daß die Oberschenkelschienen sich über je einen nach vorne innen verlaufenden Bogen der zwischen Becken und Oberschenkel an den Patienten anzulegen ist, an das zugehörige Seitenteil anschließen und diagonal von vorne oben nach hinten unten an die Innenseiten der Oberschenkel anzulegen sind, und die Spreizvorrichtung als insgesamt blattfederartige, den Damm- und Gesäßbereich des Patienten freilassende Spreizspange gestaltet ist.

Damit wird erreicht, daß auf das Kreuzbein eine nach vorne gerichtete Abstützkraft einwirkt, daß ferner bei abgespreizter Beinposition ein Druck von oben auf die Oberschenkelhalsbereiche und gleichzeitig ein Druck von unten in den Bereichen der Kniekehlen auf die Oberschenkel ausgeübt wird, wodurch ohne jeden schenkel- oder leibumspannenden Gurt der Hüftkopf jedes Hüftgelenks zur Mitte der zugehörigen Pfanne hin orientiert wird. Die

- 4 -

drei beschriebenen Stützpunkte sind infolge der blattfederartigen Gestaltung der erfindungsgemäßen Spreizspange derart
flexibel miteinander verbunden, daß sie in jeder Stellung des
Patienten den erforderlichen Korrekturdruck ausüben. Der kindliche Patient ist an den schmutzenden Körperteilen frei, kann
jede handelsübliche Windelhose tragen und alle seinem Alter
entsprechenden Bewegungen ausführen, ohne daß die Gefahr einer
den Behandlungserfolg infragestellenden Verschiebung der Spreizspange entsteht.

Die erfindungsgemäße Spreizspange ist vorzugsweise einstückig
aus Kunststoff, vorzugsweise Acryl-Butadien-Styrol, geformt,
wobei sich verschiedene Mischpolymerisate als besonders geeignet erwiesen haben.

Bei einer bevorzugten Ausführungsform der Erfindung liegen
die Längsachsen der Oberschenkelschienen im entspannten Zustand
zumindest annähernd in einer gemeinsamen, zur Symmetrieebene
der Spreizvorrichtung normalen Ebene.

Die derart ausgestaltete erfindungsgemäße Spreizspange wird
von hinten her an den Patienten angelegt, so daß die Rückenstütze
im Bereich des Kreuzbeins aufliegt. Von dort verlaufen die beiden Seitenteile in die Oberschenkelbereiche und jeder der sich
daran anschließenden Bogen verläuft zwischen Becken und Oberschenkel zur Vorderseite des Oberschenkels. Von dort verläuft
die zugehörige Oberschenkelschiene über die Vorder- und Innenseite des Oberschenkels diagonal in die Kniekehle, wo der untere
Teil des Oberschenkels von der zugehörigen Stützschale umfaßt
wird, nachdem der Oberschenkel von hinten her in die Stützschale
hineingehebelt worden ist. Das Kind ist dann nicht in der Lage,
die erfindungsgemäße Spreizspange ohne fremde Hilfe abzustreifen.

Ein Ausführungsbeispiel der Erfindung wird im folgenden anhand
schematischer Zeichnungen näher erläutert. Es zeigt:

Fig. 1 ein Kind mit Spreizspange in Rückenlage,

Fig. 2 das Kind in Bauchlage,

Fig. 3 eine Hälfte der Spreizspange in entspanntem
Zustand, von vorne und im größeren Maßstab dargestellt,

Fig. 4 die Draufsicht der in Fig.3 dargestellten Hälfte
der Spreizspange in Richtung des Pfeils IV,

Fig. 5 die Seitenansicht in Richtung des Pfeils V in

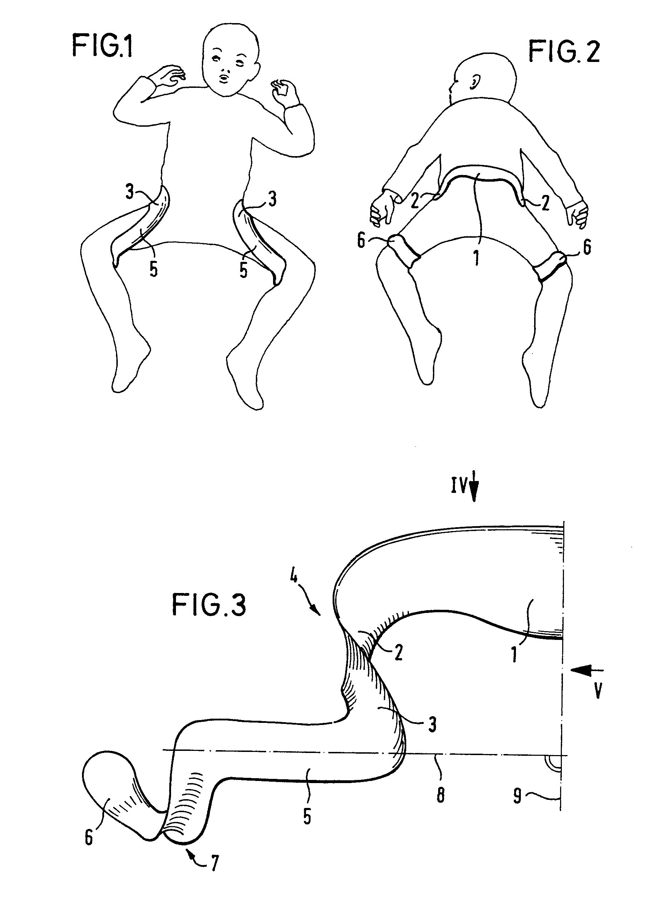

Fig.3 und

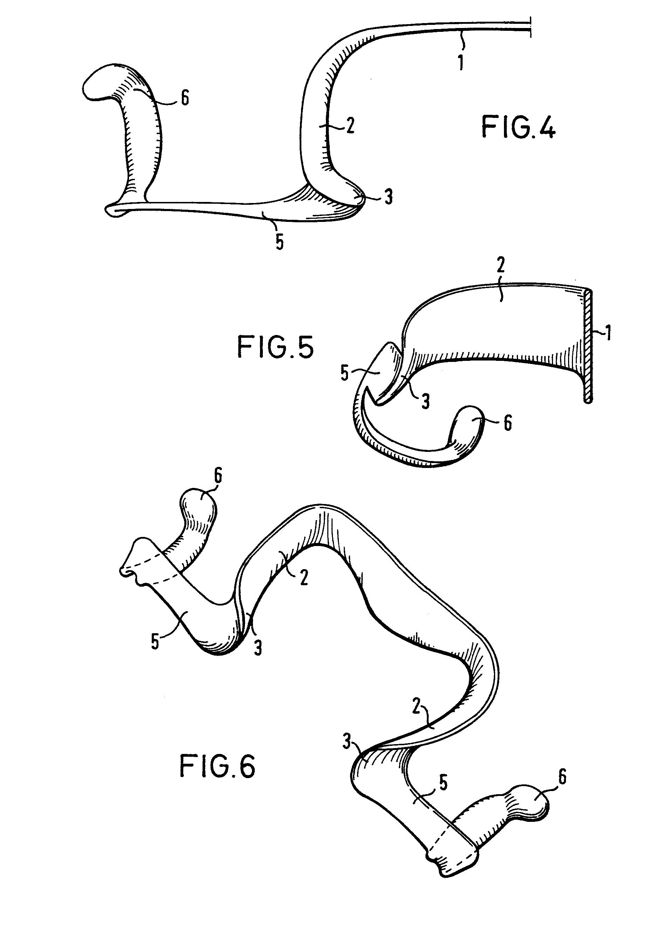

Fig. 6 eine Schrägansicht der gesamten Spreizspange.

Die Spreizvorrichtung weist eine breite Rückenabstützung 1 auf, von der sich zwei Seitenteile 2 nach vorne erstrecken. An jedes der Seitenteile schließt sich ein Bogen 3 an, der den zugehörigen Oberschenkel in abgespreizter Position in der Falte zwischen Becken und Oberschenkel überspannt, so daß er einen entsprechend dem Pfeil 4 nach unten gerichteten Druck auf das Hüftgelenk ausübt. An jeden Bogen 3 schließt sich eine Oberschenkelschiene 5 an, die in einem sanften Bogen diagonal über die Innenseite des zugehörigen Oberschenkels bis zu dessen Mitte und dann gerade bis zur Kniekehle verläuft. Am Ende jeder Oberschenkelschiene 5 ist eine Stützschale 6 ausgebildet, die entsprechend dem Pfeil 7 einen nach oben gerichteten Druck auf den Oberschenkel ausübt.

Im entspannten Zustand der erfindungsgemäßen Spreizspange liegen die Längsachsen 8 der Oberschenkelschienen 5 gemäß Fig.3 in einer zur Symmetrieebene 9 der Spreizspange normalen Ebene; dabei nehmen die Stützschalen 6 eine Stellung ein, in der ihre offene Seite nach oben weist.

PATENTANWÄLTE

WUESTHOFF-v.PECHMANN-BEHRENS-GOETZ

PROFESSIONAL REPRESENTATIVES BEFORE THE EUROPEAN PATENT OFFICE
MANDATAIRES AGRÉÉS PRÈS L'OFFICE EUROPÉEN DES BREVETS

D-8000 MÜNCHEN 90
SCHWEIGERSTRASSE 2
TELEFON: (089) 66 20 51
TELEGRAMM: PROTECTPATENT
TELEX: 524070

EP-51 861

Patentansprüche:

1. Spreizvorrichtung zum Behandeln von Hüft-Dysplasien mit einer im Bereich des Kreuzbeins an den Rücken eines Patienten anlegbaren Rückenstütze, von der sich zwei Seitenteile nach vorne erstrecken, mit denen je eine blattfederartige Oberschenkelschiene verbunden ist, an deren Ende je eine Stützschale befestigt ist, die an oder über der Kniekehle an je einen Oberschenkel des Patienten anzulegen ist, dadurch g e k e n n - z e i c h n e t , daß die Oberschenkelschienen (5) sich über je einen nach vorne innen verlaufenden Bogen (3), der zwischen Becken und Oberschenkel an den Patienten anzulegen ist, an das zugehörige Seitenteil (2) anschließen und diagonal von vorne oben nach hinten unten an die Innenseiten der Oberschenkel anzulegen sind, und die Spreizvorrichtung als insgesamt blattfederartige, den Damm- und Gesäßbereich des Patienten freilassende Spreizspange gestaltet ist.

2. Spreizvorrichtung nach Anspruch 1, dadurch g e k e n n - z e i c h n e t , daß sie einstückig aus Kunststoff geformt ist.

3. Spreizvorrichtung nach Anspruch 1 oder 2, dadurch g e - k e n n z e i c h n e t , daß die Längsachsen (8) der Oberschenkelschienen (5) im entspannten Zustand zumindest annähernd in einer gemeinsamen, zur Symmetrieebene (9) der Spreizvorrichtung normalen Ebene liegen.

5627

FIG.1

FIG.2

FIG.3

FIG.4

FIG.5

FIG.6